# EUROPÄISCHE PATENTSCHRIFT

(11) **EP 1 032 444 B1**
(45) Veröffentlichungstag und Bekanntmachung des Hinweises auf die Patenterteilung: **19.05.2004**
(21) Anmeldenummer: 97911100.2
(22) Anmeldetag: 17.11.1997
(51) Int. Cl.: A61M 5/32

(54) **NADELPENETRATIONSVORRICHTUNG**
DEVICE FOR INTRODUCING A NEEDLE
DISPOSITIF POUR FAIRE PENETRER UNE AIGUILLE

(43) Veröffentlichungstag der Anmeldung: 06.09.2000
(73) Patentinhaber: Tecpharma Licensing AG, 3401 Burgdorf (CH)
(72) Erfinder: WICH, Horst, Cathedral City, CA 92234 (US)
(74) Vertreter: Schwabe - Sandmair - Marx
(86) Internationale Anmeldenummer: PCT/CH1997/000434
(87) Internationale Veröffentlichungsnummer: WO 1999/025400

(56) Entgegenhaltungen:
- EP-A- 0 518 416
- FR-A- 1 409 793
- US-A- 2 531 267
- US-A- 2 664 086
- US-A- 4 445 510

## Beschreibung

Die Erfindung betrifft eine Nadelpenetrationsvorrichtung zum Injizieren einer flüssigen Substanz.

Bei konventionellen Injektionsvorrichtungen bekannter Art besteht häufiger das Problem, dass der Bediener sich selber nicht die Injektionsnadel in die Gewebehaut einführen kann und er daher auf Mithilfe oder eine Fachkraft angewiesen ist.

Der vorliegenden Erfindung liegt daher die Aufgabe zugrunde, eine Nadelpenetrationsvorrichtung anzugeben, durch die es möglich ist, dass dem Bediener das Einführen der Injektionsnadel in die Gewebehaut abgenommen wird und er sich mittels der konventionellen Injektionsvorrichtung die für ihn bestimmte Dosis verabreichen kann.

Die Erfindung löst die gestellte Aufgabe mit einer Nadelpenetrationsvorrichtung, welche die Merkmale des kennzeichnenden Teils des unabhängigen Anspruchs 1 aufweist.

Als Bedienelement dient eine Auslösehülse, die auf einem Durchstechnadelhalter, der die Durchstechnadel hält, längsverschiebbar angeordnet ist und mit einem in einer Anschlaghülse ebenfalls längsverschiebbaren Injektionsnadelhalter, der die Injektionsnadel hält, zusammenwirkt.

Das Einführen der Injektionsnadel in die Gewebehaut nach manuellem Auslösen der Auslösehülse erfolgt dadurch, dass der Injektionsnadelhalter zusammen mit der Injektionsnadel mittels der Kraft einer Feder selbsttätig in Richtung der Gewebehaut bewegt wird. Nach selbsttätigem Einführen der Injektionsnadel in die Gewebehaut kann sich der Bediener mittels der konventionellen Injektionsvorrichtung die zu verabreichende Dosis der flüssigen Substanz zuführen.

Die erfindungsgemässe Nadelpenetrationsvorrichtung hat den wesentlichen Vorteil, dass sie auf jede handelsübliche konventionelle Injektionsvorrichtung angekoppelt werden kann, dass durch die Nadelpenetrationsvorrichtung eine grosse Bediensicherheit hinsichtlich der Eindringtiefe der Injektionsnadel in die Gewebehaut erzielt werden kann, und dass diese Nadelpenetrationsvorrichtung in ihrem Aufbau relativ einfach und damit kostengünstig ist.

Weitere vorteilhafte Ausgestaltungen der Erfindung ergeben sich aus den Unteransprüchen.

Ein Ausführungsbeispiel der Erfindung ist in den Figuren dargestellt.

Es zeigen:
Fig. 1: einen Längsschnitt durch die erfindungsgemässe Nadelpenetrationsvorrichtung in der Ausgangsposition oder Ruhelage,
Fig. 2: einen Längsschnitt der Nadelpenetrationsvorrichtung gemäss Fig. 1 im entsicherten Zustand nach Abziehen der Sicherungskappe; und
Fig. 3: einen Längsschnitt der Nadelpenetrationsvorrichtung nach manuellem Auslösen der Auslösehülse.

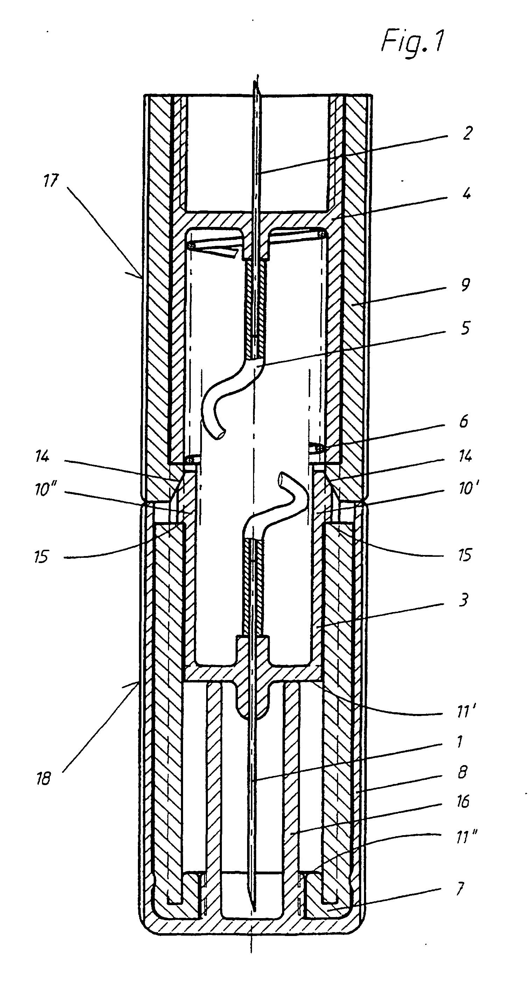

Fig. 1 zeigt die erfindungsgemässe Nadelpenetrationsvorrichtung zum Injizieren einer flüssigen Substanz. Die Nadelpenetrationsvorrichtung ist mit der konventionellen Injektionsvorrichtung 12 koppelbar, beispielsweise durch einen Gewindeverschluss oder durch eine Rast-Nocken-Aufsteckverbindung (nicht dargestellt). Über die am einen Ende der Nadelpenetrationsvorrichtung angebrachte Durchstechnadel 2 ist die Nadelpenetrationsvorrichtung mit der Penkarpule 13 der konventionellen Injektionsvorrichtung 12 in Verbindung. Über die am anderen Ende der Nadelpenetrationsvorrichtung angebrachte Injektionsnadel 1 ist die zu applizierende flüssige Substanz in die Gewebehaut injizierbar. Zwischen Durchstechnadel 2 und Injektionsnadel 1 ist die flexible Verbindung 5 für den Transport der flüssigen Substanz vorgesehen. Der Vorgang des Einführens der Injektionsnadel 1 in die Gewebehaut erfolgt nach manuellem Auslösen eines Bedienelements in Form der Auslösehülse 9 der Nadelpenetrationsvorrichtung selbsttätig, und anschliessend kann über die konventionelle Injektionsvorrichtung 12 die flüssige Substanz verabreicht werden. Die Auslösehülse 9 ist auf dem Durchstechnadelhalter 4, der die Durchstechnadel 2 hält, längsverschiebbar angeordnet und wirkt mit dem im Durchstichnadelhalter 4 sowie in der Anschlaghülse 7 ebenfalls längsverschiebbaren Injektionsnadelhalter 3, der die Injektionsnadel 1 hält, zusammen. Das Einführen der Injektionsnadel 1 in die Gewebehaut erfolgt nach manuellem Auslösen der Auslösehülse 9 mittels der Kraft der Feder 6, indem der Injektionsnadelhalter 3 mit der Injektionsnadel 1 selbsttätig gegen die Gewebehaut bewegt wird. Dabei stützt sich die Feder 6 mit ihrem einen Ende am ortsfesten Durchstechnadelhalter 4 ab und bewegt mit ihrem anderen Ende- den Injektionsnadelhalter 3 mit der Injektionsnadel 1 in Richtung der Gewebehaut, wodurch in diese die Injektionsnadel 1 selbsttätig eingeführt wird. Das Zusammenwirken er Auslösehülse 9 und dem Injektionsnadelhalter 3 beim manuellen Auslösen der Auslösehülse 9 erfolgt über die konischen Abschrägungen 14, durch die der Injektionsnadelhalter 3 im Durchmesser zusammengedrückt wird und so die Verriegelung, bestehend aus dem Anschlag 15 der Anschlaghülse 7 und den Haltenocken 10', 10'' des Injektionsnadelhaltes 3 aufgehobenwird und dass somit die Feder 6 ihre Schiebewirkung entfalten kann. Der Injektionsnadelhalter 3 wird mit der Injektionsnadel 1 nach dem manuellen Auslösen der Auslösehülse 9 durch die Kraft der Feder 6 solange in Richtung der Gewebehaut selbsttätig bewegt, bis die Stirnfläche 11' des Injektionsnadelhalters 3 an der Stirnfläche 11'' der Anschlaghülse 7 zum Anliegen kommt. Nach erfolgter Injizierung ist der Injektionsnadelhalter 3 mit der Injektionsnadel 1 mittels der die Anschlaghülse 7 umgebenden Sicherungskappe 8 manuell gegen die Kraft der Feder 6 zurückschiebbar, bis die Verriegelung, bestehend aus dem Anschlag 15 der Anschlaghülse 7 und den Haltenocken 10', 10'' des Injektionsnadelhalter 3 wieder wirksam ist. Gleichzeitig wird die Auslösehülse 9 mittels der konischen Abschrägungen 14 in Richtung der konventionellen Injektionsvorrichtung 12 durch die Sicherungskappe 8 längsverschoben, so dass die Auslösehülse 9 in die Ausgangsposition oder Ruhelage zurückbewegt wird und für eine erneute manuelle Auslösung bereit ist. Zum Zurückschieben des Injektionsnadelhalters 3 mit der Injektionsnadel 1 mittels der Sicherungskappe 8, ist letztere in ihrem Innern mit einem die Injektionsnadel 1 umgebenden Schutzzylinder 16 versehen, der zum einen die Injektionsnadel 1 schützt. Zum anderen hat der Schutzzylinder 16 die Funktion, dass er mit seinem offenen Ende an der Stirnfläche 11' des Injektionsnadelhalters 3 anliegt und somit beim Aufschieben der Sicherungskappe 8 in die Injektionsnadel 1 schützendem Zustand den Injektionsnadelhalter 3 samt Injektionsnadel sowie die Auslösehülse 9 in die Ausgangsposition zurückschiebt.

Die Funktionsweise der erfindungsgemässen Nadelpenetrationsvorrichtung ist folgende:

Die Nadelpenetrationsvorrichtung wird mit der konventionellen Injektionsvorrichtung 12 mechanisch gekoppelt durch beispielsweise. einen Gewindeverschluss oder durch eine Rast-Nocken-Aufsteckverbindung oder ähnlichem. Dabei durchsticht die Durchstechnadel 2 die Membran der Penkarpule und sorgt somit dafür, dass die zu injizierende flüssige Substanz in die Nadelpenetrations- vorrichtung fliessen kann. Der Bediener führt nun letztere an eine bevorzugte Gewebehautstelle, löst anschliessend die Auslösehülse 9 aus, indem er diese in Richtung Gewebehaut nach unten längsverschiebt, bis die Verriegelung, bestehend aus dem Anschlag 15 der Anschlaghülse 7 und den Haltenocken 10', 10'' des Injektionsnadelhalters 3 aufgehoben wird. Dadurch kann die Feder 6 ihre Kraft entfalten und bewegt den Injektionsnadelhalter 3 mit der Injektionsnadel 1 solange in Richtung der Gewebehaut selbsttätig, bis die Stirnfläche 11' des Injektionsnadelhalters 3 an der Stirnfläche 11'' der Anschlaghülse 7 zum Anliegen kommt. Dadurch ist die Injektionsnadel 1 selbsttätig in die Gewebehaut eingeführt worden. Jetzt kann der Bediener sich mittels der konventionellen Injektionsvorrichtung 12 die zu verabreichende Dosis an flüssiger Substanz applizieren. Nach erfolgter Injizierung ist der Injektionsnadelhalter 3 mit der Injektionsnadel 1 mittels Sicherungskappe 8 manuell gegen die Kraft der Feder 6 zurückschiebbar. Die Verriegelung wird wieder wirksam und gleichzeitig kehrt die Ausläsehülse 9 in ihre Ausgangsposition oder Ruhelage zurück.

Der Durchstechnadelhalter 3 kann im Bereich der konischen Abschrägungen 14 vorzugsweise geschlitzt sein zur Elastizitätsverbesserung, wenn er beim Zusammenwirken mit der Auslösehülse 9 beim Auslösevorgang zusammengedrückt wird, um die Verriegelung aufzuheben.

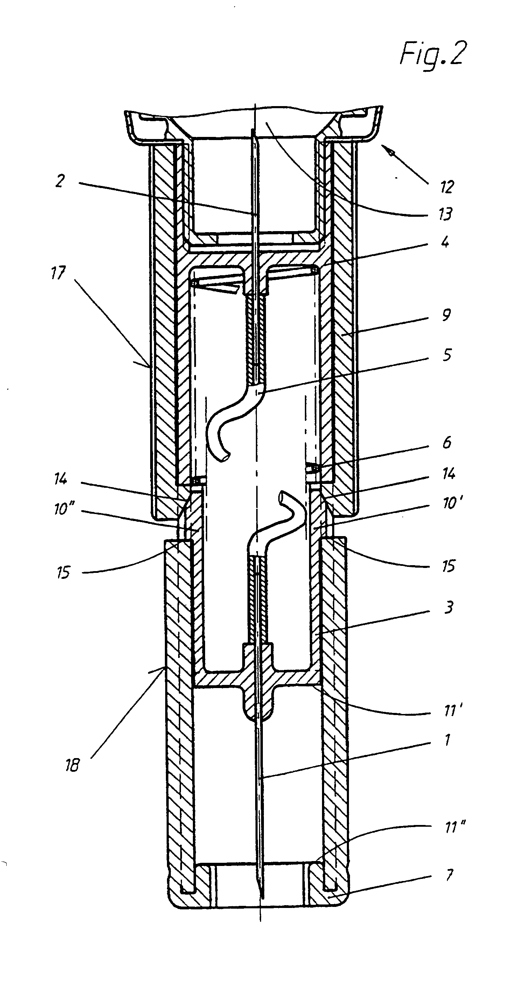

Die Fig. 2 zeigt einen Längsschnitt der Nadelpenetrationsvorrichtung im entsicherten Zustand nach Abziehen der Sicherungskappe 8. Bis auf diese Sicherungskappe 8 ist Fig. 2 im wesentlichen identisch mit Fig. 1. Die Bezugszeichen sind in beiden Figuren identisch.

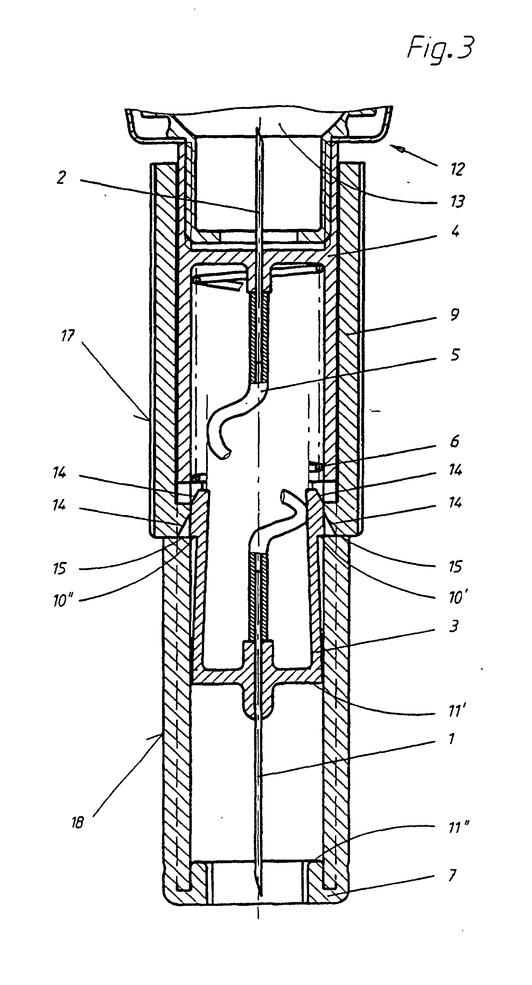

Fig. 3 zeigt einen Längsschnitt der Nadelpenetrationsvorrichtung nach manuellem Auslösen der Auslösehülse 9. Diese Figur zeigt, wie unmittelbar nach Aufheben der Verriegelung, bestehend aus dem Anschlag 15 der Anschlaghülse 7 und den Haltenocken 10', 10'' des Injektionsnadelhalters 3, durch das Zusammenwirken der konischen Abschrägungen 14 von Injektionsnadelhalter 3 und Auslösehülse 9 der Injektionsnadelhalter 3 federnd zusammengedrückt wird, damit die Feder 6 ihre Kraft entfalten kann, und der selbsttätige Einführvorgang der Injektionsnadel 1 in die Gewebehaut beginnen kann.

Die erfindungsgemässe Nadelpenetrationsvorrichtung ist anwendbar zum Verabreichen von vorzugsweise Medikamenten. Sie kann darüberhinaus überall dort angewendet werden, wo eine flüssige Substanz in einen Körper einzubringen ist und die Injektionsnadel selbsttätig eingeführt werden soll.

## Patentansprüche

1. Nadelpenetrationsvorrichtung zur Injizierung einer flüssigen Substanz, umfassend
einen hinteren longitudinalen Teil (17) mit einer Durchstechnadel (2), welcher mit einer konventionellen Injektionsvorrichtung (12) koppelbar ist;
und ein Bedienelement (9) aufweist zum manuellen Auslösen einer in einem vorderen longitudinalen Teil (18) längsverchiebbar angeordneten Injektionsnadel (1) zur Applikation der flüssigen Substanz in die Gewebehaut; und
eine flexible Verbindung (5) zwischen der Durchstechnadel (2) und der Injektionsnadel (1) für den Transport der flüssigen Substanz.

2. Nadelpenetrationsvorrichtung nach Anspruch 1, **dadurch gekennzeichnet, dass** das Bedienelement eine Auslösehülse (9) ist, die auf einem Durchstechnadelhalter (4), der die Durchstechnadel (2) hält, längsverschiebbar angeordnet ist und mit einem in einer Anschlaghülse (7) ebenfalls längsverschiebbaren Injektionsnadelhalter (3), der die Injektionsnadel (1) hält, zusammenwirkt.

3. Nadelpenetrationsvorrichtung nach Anspruch 1 - 2, **dadurch gekennzeichnet, dass** das Einführen der Injektionsnadel (1) in die Gewebehaut nach manuellem Auslösen der Auslösehülse (9) der Injektionsnadelhalter (3) mit der Injektionsnadel (1) mittels der Kraft der Feder (6) erfolgt.

4. Nadelpenetrationsvorrichtung nach einem der Ansprüche 1 bis 3, **dadurch gekennzeichnet, dass** sich die Feder (6) mit ihrem einen Ende am ortsfesten Durchstechnadelhalter (4) abstützt und mit ihrem anderen Ende den Injektionsnadelhalter (3) mit der Injektionsnadel (1) in Richtung der Gewebehaut bewegt und in diese die Injektionsnadel (1) selbsttätig einführt.

5. Nadelpenetrationsvorrichtung nach einem der Ansprüche 1 bis 4, **dadurch gekennzeichnet, dass** das Zusammenwirken von Auslösehülse (9) und Injektionsnadelhalter (3) beim manuellen Auslösen der Auslösehülse (9) über konische Abschrägungen (14) erfolgt, durch die der Injektionsnadelhalter (3) im Durchmesser zusammengedrückt wird und so eine Verriegelung, bestehend aus einem Anschlag (15) der Anschlaghülse (7) und Haltenocken (10', 10'') des Injektionsnadelhalters (3) aufgehoben wird, so dass die Feder (6) ihre Schiebewirkung entfalten kann.

6. Nadelpenetrationsvorrichtung nach einem der Ansprüche 1 - 5, **dadurch gekennzeichnet, dass** der Injektionsnadelhalter (3) mit der Injektionsnadel (1) nach manuellem Auslösen der Auslösehülse (9) durch die Kraft der Feder (6) solange in Richtung der Gewebehaut selbsttätig bewegt wird, bis eine Stirnfläche (11') des Injektionsnadelhalters (3) an einer Stirnfläche (11'') der Anschlaghülse (7) zum Anliegen kommt.

7. Nadelpenetrationsvorrichtung nach einem der Ansprüche 1 - 6, **dadurch gekennzeichnet, dass** nach erfolgter Injizierung der Injektionsnadelhalter (3) mit der Injektionsnadel (1) mittels einer die Anschlaghülse (7) umgebenden Sicherungskappe (8) manuell gegen die Kraft der Feder (6) zurückschiebbar ist, bis die Verriegelung, bestehend aus dem Anschlag (15) der Anschlaghülse (7) und den Haltenocken (10', 10'') des Injektionsnadelhalters (3) wieder wirksam ist und gleichzeitig die Auslösehülse (9) vermittelst der konischen Abschrägungen (14) in Richtung der konventionellen Injektionsvorrichtung (12) längsverschiebbar ist, und die Auslösehülse (9) für eine erneute manuelle Auslösung bereit ist.

8. Nadelpenetrationsvorrichtung nach einem der Ansprüche 1 bis 7, **dadurch gekennzeichnet, dass** die Ankoppelung der Nadelpenetrationsvorrichtung an die konventionelle Injektionsvorrichtung (12) mittels eines Gewindeverschlusses oder einer Rast-Nocken-Aufsteckerbindung erfolgt.

9. Nadelpenetrationsvorrichtung nach einem der Ansprüche 1 bis 8, **dadurch gekennzeichnet, dass** die Sicherungskappe (8) in ihrem Innern einen die Injektionsnadel (1) umgebenden Schutzzylinder (16) aufweist.

## Claims

1. A needle penetration device for injecting a liquid substance, comprising: a rear longitudinal portion (17) comprising a piercing needle (2), which may be coupled to a conventional injection device (12) and comprises an operating element (9) for manually triggering an injection needle (1) arranged in a front longitudinal portion (18) such that it may be shifted longitudinally, for administering the liquid substance into the tissue skin; and a flexible connection (5) between the piercing needle (2) and the injection needle (1), for transporting the liquid substance.

2. The needle penetration device as set forth in claim 1, **characterised in that** the operating element is a triggering sleeve (9) which is arranged, such that it may be shifted longitudinally, on a piercing needle holder (4) which holds the piercing needle (2), and cooperates with an injection needle holder (3) which is likewise arranged in a stopper sleeve (7) such that it may be shifted longitudinally and holds the injection needle (1).

3. The needle penetration device as set forth in claims 1 to 2, **characterised in that** the injection needle (1) is introduced into the tissue skin by means of the force of a spring (6), once the triggering sleeve (9) of the injection needle holder (3) comprising the injection needle (1) has been manually triggered.

4. The needle penetration device as set forth in any one of claims 1 to 3, **characterised in that** the spring (6) is supported via one end on the positionally fixed piercing needle holder (4) and via its other end moves the injection needle holder (3) comprising the injection needle (1) towards the tissue skin and automatically introduces the injection needle (1) into the tissue skin.

5. The needle penetration device as set forth in any one of claims 1 to 4, **characterised in that** the triggering sleeve (9) and the injection needle holder (3) co-operate via conical chamfers (14) when the triggering sleeve (9) is manually triggered, said chamfers (14) pressing the injection needle holder (3) together in its diameter and thus lifting a block consisting of a stopper (15) of the stopper sleeve (7) and holding cams (10', 10") of the injection needle holder (3), such that the spring (6) can deploy its pushing effect.

6. The needle penetration device as set forth in any one of claims 1 to 5, **characterised in that** the injection needle holder (3) comprising the injection needle (1) is automatically moved by the force of the spring (6) towards the tissue skin, once the triggering sleeve (9) has been manually triggered, until a facing surface (11') of the injection needle holder (3) comes to rest on a facing surface (11") of the stopper sleeve (7).

7. The needle penetration device as set forth in any one of claims 1 to 6, **characterised in that** once the injection has been performed, the injection needle holder (3) comprising the injection needle (1) may be manually pushed back against the force of the spring (6) by means of a safety cap (8) surrounding the stopper sleeve (7), until the block consisting of the stopper (15) of the stopper sleeve (7) and the holding cams (10', 10") of the injection needle holder (3) is active again, and **in that** the triggering sleeve (9) may simultaneously be shifted longitudinally towards the conventional injection device (12) by means of the conical chamfers (14), and the triggering sleeve (9) is ready to be manually triggered again.

8. The needle penetration device as set forth in any one of claims 1 to 7, **characterised in that** the needle penetration device is coupled to the conventional injection device (12) by means of a threaded lock or a locking cam plug connection.

9. The needle penetration device as set forth in any one of claims 1 to 8, **characterised in that** the safety cap (8) comprises, in its interior, a protective cylinder (16) surrounding the injection needle (1).

## Revendications

1. Dispositif de pénétration d'aiguille pour l'injection d'une substance liquide,
comprenant
une partie longitudinale arrière (17) avec une aiguille de perforation (2), qui peut être couplée avec un dispositif d'injection (12) classique,
et présente un élément de commande (9) pour le déclenchement manuel d'une aiguille d'injection (1) disposée dans une partie longitudinale avant (18) de façon coulissante longitudinalement pour l'application de la substance liquide dans le tissu de la peau et
une liaison flexible (5) entre l'aiguille de perforation (2) et l'aiguille d'injection (1) pour le transport de la substance liquide.

2. Dispositif de pénétration d'aiguille selon la revendication 1, **caractérisé en ce que** l'élément de commande est une douille de déclenchement (9) qui est disposée sur un support d'aiguille de perforation (4), qui maintient l'aiguille de perforation (2), de façon coulissante longitudinalement et coopère avec un support d'aiguille d'injection (3) également coulissant dans le sens longitudinal dans une douille de butée (7), lequel support maintient l'aiguille d'injection (1).

3. Dispositif de pénétration d'aiguille selon les revendications 1-2, **caractérisé en ce que** l'introduction de l'aiguille d'injection (1) dans le tissu de la peau après le déclenchement manuel de la douille de déclenchement (9) du support d'aiguille d'injection (3) s'effectue avec l'aiguille d'injection (1) au moyen de la force du ressort (6).

4. Dispositif de pénétration d'aiguille selon l'une quelconque des revendications 1 à 3, **caractérisé en ce que** le ressort (6) s'appuie avec l'une de ses extrémités sur le support fixe d'aiguille de perforation (4) et déplace avec son autre extrémité le support d'aiguille d'injection (3) avec l'aiguille d'injection (1) en direction du tissu de la peau et introduit automatiquement l'aiguille d'injection (1) dans celui-ci.

5. Dispositif de pénétration d'aiguille selon l'une quelconque des revendications 1 à 4, **caractérisé en ce que** l'action conjuguée de la douille de déclenchement (9) et du support d'aiguille d'injection (3) s'effectue lors du déclenchement manuel de la douille de déclenchement (9) au moyen de chanfreins coniques (14), par lesquels le support d'aiguille d'injection (3) est comprimé en diamètre et un verrouillage comprenant une butée (15) de la douille de butée (7) et des cames de retenue (10', 10") du support d'aiguille d'injection (3) est supprimé, de sorte que le ressort (6) peut déployer son effet de poussée.

6. Dispositif de pénétration d'aiguille selon l'une quelconque des revendications 1 à 5, **caractérisé en ce que** le support d'aiguille d'injection (3) avec l'aiguille d'injection (1) est déplacé automatiquement après le déclenchement manuel de la douille de déclenchement (9) par la force du ressort (6) en direction du tissu de la peau jusqu'à ce qu'une face frontale (11') du support d'aiguille d'injection (3) s'appuie sur une face frontale (11") de la douille de butée (7).

7. Dispositif de pénétration d'aiguille selon l'une quelconque des revendications 1 à 6, **caractérisé en ce que**, une fois l'injection effectuée, le support d'aiguille d'injection (3) avec l'aiguille d'injection (1) peut être reculé manuellement contre la force du ressort (6) au moyen d'un capuchon de sécurité (8) entourant la douille de butée (7) jusqu'à ce que le verrouillage comprenant la butée (15) de la douille de butée (7) et les cames de retenue (10', 10") du support d'aiguille d'injection (3) devienne à nouveau actif, et que dans le même temps la douille de déclenchement (9) puisse coulisser longitudinalement au moyen des chanfreins (14) coniques en direction du dispositif d'injection (12) classique(12), et que la douille de déclenchement (9) soit prête pour un nouveau déclenchement manuel.

8. Dispositif de pénétration d'aiguille selon l'une quelconque des revendications 1 à 7, **caractérisé en ce que** le raccordement du dispositif de pénétration d'aiguille au dispositif d'injection classique (12) s'effectue au moyen d'une fermeture filetée ou d'une liaison d'emboîtement cran-came.

9. Dispositif de pénétration d'aiguille selon l'une quelconque des revendications 1 à 8, **caractérisé en ce que** le capuchon de sécurité (8) présente dans son intérieur un cylindre de protection (16) entourant l'aiguille d'injection (1).
